(19) Europäisches Patentamt
European Patent Office
Office européen des brevets

(11) Veröffentlichungsnummer: **0 010 652**
**B1**

(12) # EUROPÄISCHE PATENTSCHRIFT

(45) Veröffentlichungstag der Patentschrift: **18.05.83**

(21) Anmeldenummer: **79103864.9**

(22) Anmeldetag: **09.10.79**

(51) Int. Cl.³: **C 07 D 263/34,** D 06 L 3/12, G 03 G 5/06

(54) **Neue 4-Chloroxazole, Verfahren zu deren Herstellung und deren Verwendung in photoleitenden Schichten in der Elektrophotographie und als optische Aufheller.**

(30) Priorität: **12.10.78 DE 2844394**

(43) Veröffentlichungstag der Anmeldung:
**14.05.80 Patentblatt 80/10**

(45) Bekanntmachung des Hinweises auf die Patenterteilung:
**18.05.83 Patentblatt 83/20**

(84) Benannte Vertragsstaaten:
**AT BE CH DE FR IT NL SE**

(56) Entgegenhaltungen:
**DE - A - 1 445 545**
**DE - C - 849 694**
**DE - C - 869 490**
**US - A - 3 574 228**
**US - A - 3 862 169**

(73) Patentinhaber: **HOECHST AKTIENGESELLSCHAFT**
**Postfach 80 03 20**
**D-6230 Frankfurt/Main 80 (DE)**

(72) Erfinder: **Bosse, Dieter, Dr.**
**Berliner Strasse 12**
**D-6238 Hofheim am Taunus (DE)**
Erfinder: **Günther, Dieter, Dr.**
**Nachtigallenweg 1A**
**D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Neue 4-Chloroxazole, Verfahren zu deren Herstellung und deren Verwendung in photoleitenden Schichten in der Elektrophotographie und als optische Aufheller

Aus der Literatur sind bereits einige 4-Chloroxazole und Verfahren zu deren Herstellung bekannt. So ist 4 - Chlor - 2,5 - diphenyl - oxazol durch thermische Umlagerung von N - Benzoloyl - 2,5 - dichlor - 3 - phenylaziridin darstellbar [s. H. E. Zaugg und R. W. DeNet, J. Org. Chem. *36*, 1937 (1971) und US—PS 3 819 356] Oder durch Erhitzen von N-Benzoylaminoacetophenon mit einem Überschuß Phosphorpentachlorid auf 170°C [s. S. Gabriel, Ber. d. Chem. Ges. *43*, 134 (1910)]. Ferner ist bekannt, daß sich bei der Umsetzung einiger aromatischer Aldehyde mit Benzoylcyanid oder 4-Nitrobenzoylcyanid bei 0°C in HCl-gesättigtem absoluten Diethylether 4 - Chlor - 2,5 - diaryl - oxazol - hydrochloride bilden, die bei der wäßrigen Aufarbeitung die freien 4 - Chlor - 2,5 - diaryl - oxazole liefern [s. M. Davis et al., J., Heterocycl. Chem. *14*, 317 (1977)].

Gegenstand der Erfindung sind neue 4-Chloroxazole der allgemeinen Formel I bzw. II

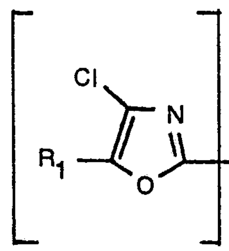
(I)

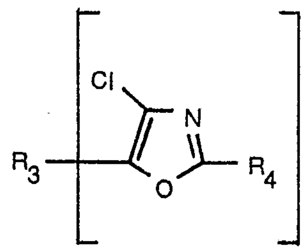
(II)

wobei n 1 oder 2 ist, im Fall n=1

$R_1$, $R_2$, $R_3$ und $R_4$ gleich oder verschieden sind und Phenyl oder Naphthyl, die durch eine bis drei Gruppen aus der Reihe Halogen, Cyano, Nitro, Trifluoromethyl, Amino, mono oder di-1—2 C-Alkylamino, 1—4 C-Alkyl, 1—2 C-Alkoxy, Hydroxy, Carbo-1—3 C-alkoxy, Phenoxy, Styryl oder Chlorstyryl substituiert sein können, oder Pyrenyl oder einen N, O oder S enthaltenden 5- oder 6-gliedrigen Heterocyclus, der durch Chlor oder Methoxy-Gruppen substituiert sein oder einen ankondensierten Benzolring tragen kann, bedeuten, $R_2$ und $R_4$ zusätzlich $C_1$—$C_{12}$-Alkyl, und im Fall n=2 $R_1$ und $R_4$ die gleiche Bedeutung haben wie oben für den Fall n=1 und $R_2$ und $R_3$ Phenylen oder Naphthylen bedeuten.

Bevorzugt sind solche Verbindungen der Formeln I bzw. II, wobei im Fall n=1 $R_1$, $R_2$, $R_3$ und $R_4$ Phenyl oder Naphthyl, die durch eine bis drei Gruppen aus der Reihe Fluor, Chlor oder Brom, Cyano, Nitro, Trifluoromethyl, Amino, mono- oder di-1—2 C-Alkylamino, 1—4 C-Alkyl, 1—2 C-Alkoxy, Hydroxy, Carbo-1—3 C-alkoxy, Phenoxy, Styryl substituiert sein können, oder Furanyl, Benzofuranyl, Thienyl, Pyrimidyl, Benzthienyl oder Benzoxazolyl, die durch Chlor oder Methoxy, substituiert sein können, bedeuten und im Fall n=2 $R_1$ und $R_4$ die gleiche Bedeutung haben, wie zuvor für n=1 angegeben und $R_2$ und $R_3$ Phenylen oder Naphthylen bedeuten.

Insbesondere bevorzugt sind Verbindungen der Formel I bzw. II, wobei n=1 ist und $R_1$, $R_2$, $R_3$ und $R_4$ Aminophenyl, Di-1—2 C-alkylaminophenyl, Fluor-, Chlor- oder Bromphenyl, Trifluormethylphenyl oder 1—2 C-Alkoxyphenyl bedeuten.

Solche Verbindungen der Formel I, bei denen n=1 ist, $R_1$ Phenyl und $R_2$ Phenyl, p-Methoxyphenyl, p-Methylphenyl, p-Nitrophenyl, p-Hydroxyphenyl oder p-Ethylphenyl bedeutet oder n=1 ist, $R_1$ p-Nitrophenyl und $R_2$ Phenyl bedeutet, werden nicht beansprucht. Bei der Styryl- bzw. Chlorstyrylgruppe, die als mögliche Substituenten für die Phenyl- und Naphtylgruppe genannt sind, handelt es sich um die Gruppen der Formeln

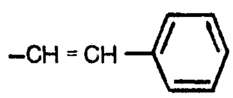
und
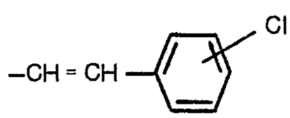

Die Furan-, Benzofuran-, Thiophen- oder Benzothiophen- Substituenten können ganz oder teilweise hydriert sein, d.h. in diesem Fall kommt als Rest $R_2$ auch beispielsweise Tetrahydrofuran in Frage.

Die Verbindungen der Formeln I bzw. II werden hergestellt, indem man ein Benzoylcyanid der allgemeinen Formel III

$$R_1 — COCN \quad \text{bzw.} \quad R_3 — (COCN)_n \qquad \text{(III)}$$

mit einem Aldehyd der allgemeinen Formel IV

$$R_2 — (CHO)_n \quad \text{bzw.} \quad R_4 — CHO \qquad \text{(IV)}$$

in Gegenwart von Chlorwasserstoff umsetzt und aus dem dabei entstandenen Oxazol-Hydrochlorid durch Behandeln mit Wasser die 4-chloroxazole der Formel I bzw. II freisetzt.

Als Benzoylcyanide der allgemeinen Formel $R_1$—$(COCN)_n$ kommen Verbindungen wie z.B. Benzoylcyanid, Chlorbenzoylcyanide, Nitrobenzoylcyanide, Methylbenzoylcyanide, Terephthaloylcyanid, Naphthoylcyanide in Betracht, die nach bekannten Literaturmethoden [vgl. Angew, Chem. *68*, 425 gg. (1956)] dargestellt werden können. Als Aldehyde R—$(CHO)_n$ kommen z.B. in Frage; Chlorbenzaldehyde, Fluorbenzaldehyde, Anisaldehyde, Phenoxybenzaldehyd, Carbomethoxybenzaldehyde, Tolylaldehyde, Naphthaldehyde, Pyrenaldehyde, Stilbenaldehyde, Dimethylaminobenzaldehyde, Thiophenaldehyde, Benzofuranaldehyde, Terephthalaldehyd oder Pyrimidinaldehyde. Sie sind nach bekannten Methoden zugänglich. Die Umsetzung erfolgt in einem inerten organischen Lösungsmittel und in Gegenwart von HCl. Brauchbare und gegenüber den Reaktanden inerte Lösungsmittel sind aliphatische und cycloaliphatische Ether wie Diethylether, Diethylenglykoldimethylether, Dimethoxyethan, Diisopropylether, Tetrahydrofuran oder Dioxan. Das erfindungsgemäße Verfahren kann in einem relativ breiten Temperaturintervall durchgeführt werden. Allgemein wird in einem Temperaturbereich von −20°C bis +30°C, vorzugsweise zwischen 0°C und +20°C gearbeitet. Die Reaktionsdauer liegt zwischen wenigen Stunden und einigen Tagen, im allgemeinen bei 12—18 Stunden. Das Verhältnis von Lösungsmittel zur Summe der Reaktionspartner kann in einem relativ weiten Bereich variiert werden, allgemein wird ein Gewichtsverhältnis von 15:1 bis 2:1 von Lösungsmittel zu Summe von Aldehyd und Benzoylcyanid bevorzugt. Im allgemeinen wird derartig gearbeitet, daß die Reaktionspartner in dem betreffenden Lösungsmittel gelöst werden und die entstandene Lösung anschließend bei −20°C bis +5°C mit Chlorwasserstoff gesättigt wird, Die auftretende Reaktionswärme wird im allgemeinen durch Außenkühlung abgeführt. Bei der Reaktion ist es zweckmäßig, die beiden Reaktionspartner in annähernd stöchiometrischem Verhältnis einzusetzen, jedoch kann der verwendete Aldehyd auch im Überschuß vorliegen.

Bei dieser Reaktion erhält man zunächst das Hydrochlorid der Verbindung der Formel I bzw. II. Um aus diesem Hydrochlorid das freie 4-Chloroxazol zu gewinnen, hydrolysiert man das Hydrochlorid durch Behandeln mit Wasser. Dies geschieht etwa in der Weise, daß man den gebildeten Hydrochlorid-Niederschlag von der Reaktionsmischung durch Filtration mit oder ohne Druck abtrennt und aus diesen Niederschlag anschließend durch Versetzen mit Wasser und/oder Basen die 4-Chlor-1,3-oxazole freisetzt.

Es ist auch möglich, das gesamte Reaktionsgemisch auf Eis zu geben, ggf. durch Zusatz von Basen alkalisch zu stellen, das freigesetzte Oxazol abzusaugen bzw. bei flüssigen Reaktionsprodukten mit nicht wassermischbaren organischen Lösungsmitteln zu extrahieren, und anschließend das Lösungsmittel im Vakuum zu entfernen. Eine weitere Reinigung der so erhaltenen 4-Chlor-oxazole ist an sich nicht erforderlich, kann aber wenn es erwünscht ist nach bekannten Methoden, z.B. Umkristallisieren oder Destillieren erfolgen. Die Ausbeute ist nahezu quantitativ.

Die oben beschriebenen neuen 4-Chloroxazole fluoreszieren in gelöstem Zustand. Sie eignen sich deshalb zum optischen Aufhellen der verschiedensten synthetischen, halbsynthetischen oder natürlichen organischen Materialien, insbesondere zum Aufhellen von nativen oder synthetischen Fasern nach den hierfür bekannten Verfahren. Weiter zeigen diejenigen 4-Chloroxazole, die als Substituenten eine Amino- oder Dialkylaminogruppe enthalten, sehr gute photoleitende Eigenschaften. Sie können deshalb zur Herstellung von photoleitenden Schichten in der Elektrophotographie verwendet werden.

In den folgenden Beispielen sind Prozente immer Gewichtsprozente, Schmelz- und Siedepunkte sind, sofern nicht anders vermerkt, unkorrigiert:

### Beispiel 1

131,14 g (1.0 Mol) Benzoylcyanid und 180,68 g (1.1 Mol) 4-Methoxycarbonyl-benzaldehyd in 500 ml Tetrahydrofuran werden unter Rühren und Kühlen auf 0°C mit Chlorwasserstoff-Gas gesättigt. Man läßt auf +3°C erwärmen und beläßt das Reaktionsgemisch 12 Stunden bei dieser Temperatur. Der entstandene gelbe Kristallbrei wird auf 1,5 kg Eis gegeben und der Niederschlag anschließend abgesaugt, mit Wasser gewaschen und bei 50°C i.V. getrocknet.

Ausbeute: 279,52 g (89,1% d.Th.
hellgelbe Kristalle
Fp. 145—46°C (Ethanol/DMF)

Analyse ($C_{17}H_{12}ClNO_3$):
ber.: C, 65,1%; H 3,9%; Cl 11,3%; N 4,5%
gef.: C 65,1%; H 4,0%; Cl 11,5%; N 4,4%

Ultraviolett-Absorptionsmaximum: 330 nm (in DMF)

Beispiel 2

13,11 g (0.1 Mol) Benzoylcyanid und 18,06 g (0.11 Mol) 4-Methoxycarbonyl-benaldehyd wurde wie unter 1 beschrieben umgesetzt, jedoch wurden 100 ml trockener Diethylether als Lösungsmittel verwendet.

Ausbeute: 29,27 g 4-Chlor-2-(4-methoxycarbonylphenyl-5-phenyl-oxazol (93,3% d.Th.)
hellgelbe Kristalle
Fp.: 145—45°C (Ethanol/DMF)

Beispiel 3

13,11 g (0,1 Mol) Benzoylcyanid und 13,22 g (0.11 Mol) p-Toluylaldehyd werden, wie unter Beispiel 1, beschrieben, umgesetzt, jedoch werden als Lösungsmittel 100 ml Diethylenglykoldimethylether verwandt.

Ausbeute: 23,33 g 4-Chlor-2-(4-methylphenyl)-5-phenyloxazol
(86,4% d.Th.)
farblose Kristalle
Fp.: 95—96°C (Methanol)

Beispiel 4

13,41 g (0.1 Mol) Terephthaldialdehyd und 26,23 g (0,2 Mol) Benzoylcyanid in 130 ml THF werden, wie unter Beispiel 1 beschrieben, umgesetzt.

Ausbeute: 23,2 g (54% d.Th.)
Bis-1,4-(4-Chlor-5-phenyl-2-oxazolyl)-benzol
hellgelbe Kristalle
Fp.: 245—46°C (DMF)

Analyse ($C_{24}H_{14}Cl_2N_2O_2$):
ber.: C 66,5%; H 3,3%; N 6,5%; Cl 16,4%
gef.: C 66,2%; H 3,3%; N 6,5%; Cl 16,8%

Ultraviolett-Absorptionsmaximum: 368 nm
(gemessen in Dimethylformamid)

Analog Beispiel 1 werden folgende Verbindungen der allgemeinen Formel

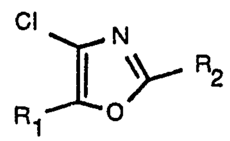

dargestellt.

| Beispiel Nr. | $R_1$ | $R_2$ | Fp. |
|---|---|---|---|
| 5 | 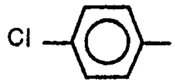 | 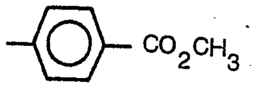 | 168 °C (Ethanol /DMF) |
| 6 | 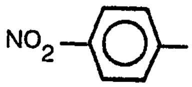 | 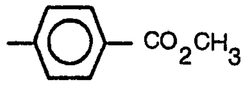 | 242 °C (DMF) |
| 7 | 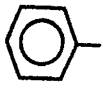 | 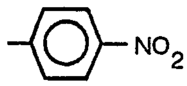 | 190 °C (DMF) |
| 8 | 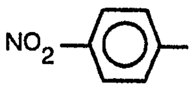 | 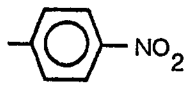 | 244 °C (DMF) |
| 9 | 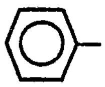 | 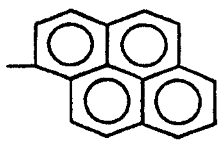 | 186 °C (DMF) |
| 10 | 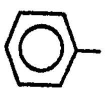 | 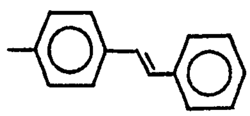 | 147 °C (DMF) |
| 11 | 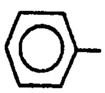 | 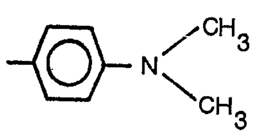 | 132 °C (Ethanol) |
| 12 | 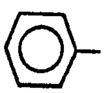 | 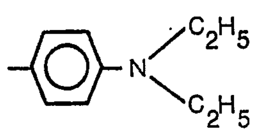 | 110 °C (Ethanol) |
| 13 | 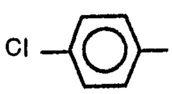 | 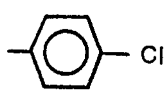 | 164 °C (DMF) |
| 14 | 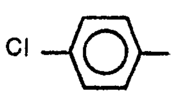 | 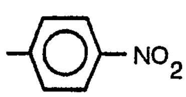 | 205 °C (n-Butanol /DMF) |

| Beispiel Nr. | $R_1$ | $R_2$ | Fp. |
|---|---|---|---|
| 15 | 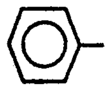 | 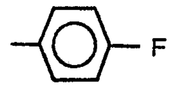 | 128 °C (Ethanol) |
| 16 | 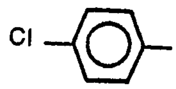 | 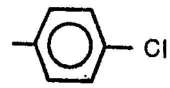 | 164 °C (DMF) |
| 17 | 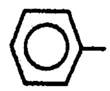 | 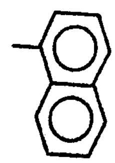 | 115 °C (Ethanol) |
| 18 | 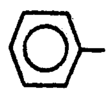 | 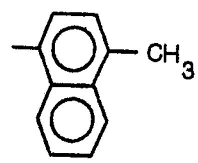 | 146 °C (Ethanol / DMF) |
| 19 | 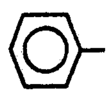 | 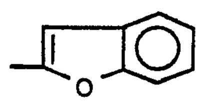 | 146 °C (Ethanol / DMF) |
| 20 | 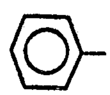 | 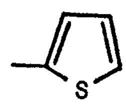 | 87 °C |
| 21 | 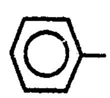 | 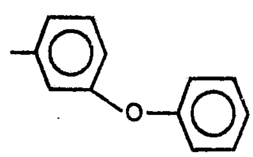 | 81 °C (Ethanol) |
| 22 | 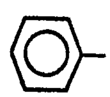 | 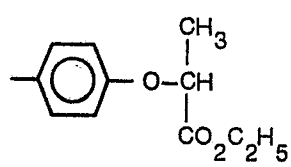 | 122 °C (Ethanol) |
| 23 | 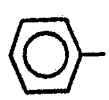 | $-C_2H_5$ | – 1) |

| Beispiel Nr. | $R_1$ | $R_2$ | Fp. |
|---|---|---|---|
| 24 | 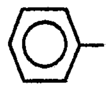 | $-CH(CH_3)_2$ | – 2) |
| 25 | 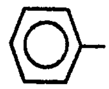 | $-C(CH_3)_3$ | – 3) |
| 26 | 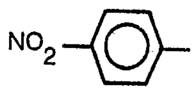 | $-C_2H_5$ | 99 °C |
| 27 | 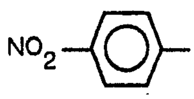 | $-C(CH_3)_3$ | 114 °C |

1) $Kp_{(0,27\ mbar)}$ = 93 –95 °C

2) $Kp_{(0,08\ mbar)}$ = 90 –93 °C

3) $Kp_{(1,07\ mbar)}$ = 106 °C

| Beispiel Nr. | $R_1$ | $R_2$ | Fp. |
|---|---|---|---|
| 28 | 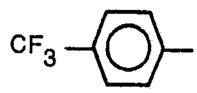 | 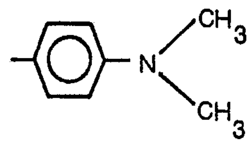 | 157 °C (EtoH) |
| 29 | 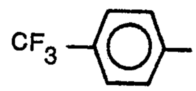 | 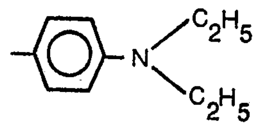 | 126 °C (EtoH) |
| 30 | 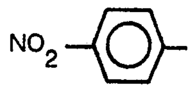 | 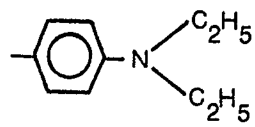 | 165 °C (EtoH / DMF) |
| 31 | 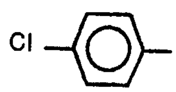 | 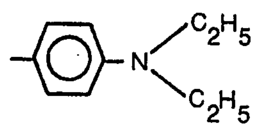 | 159 °C (EtoH / DMF) |
| 32 | 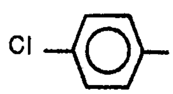 | 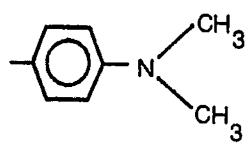 | 162 °C (EtoH / DMF) |
| 33 | 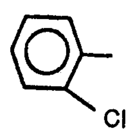 | 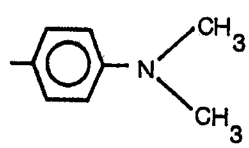 | 138 °C (MeoH) |
| 34 | 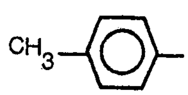 | 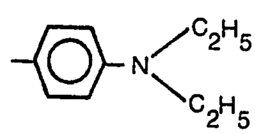 | 134 °C (EtoH) |
| 35 | 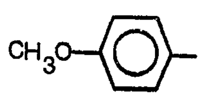 | 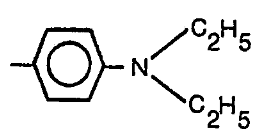 | 104 °C (EtoH) |
| 36 | 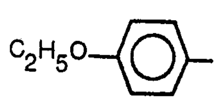 | 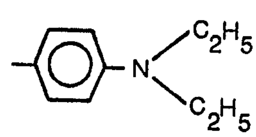 | 90 °C (EtoH) |
| 37 | 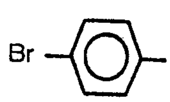 | 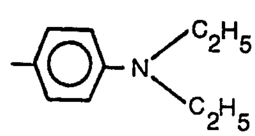 | 148 °C (EtoH / DMF) |

| Beispiel Nr. | $R_1$ | $R_2$ | Fp. |
|---|---|---|---|
| 38 | 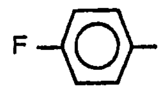 | 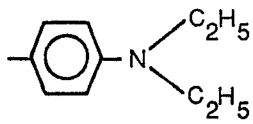 | 120 °C (EtoH) |
| 39 | 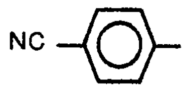 | 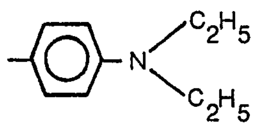 | 180 °C (EtoH / DMF) |
| 40 | 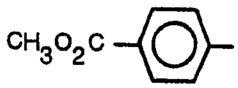 | 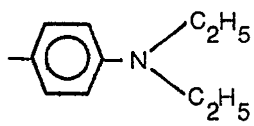 | 147 °C |
| 41 | 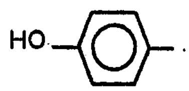 | 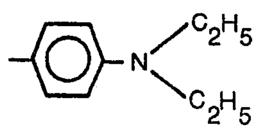 | 212 °C (MeOH) |
| 42 | 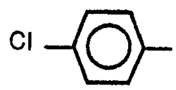 | 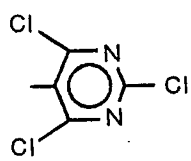 | 126 °C (EtoH) |
| 43 | 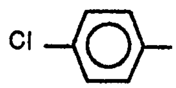 | 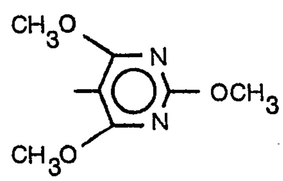 | 162 °C (EtoH) |
| 44 | 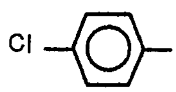 | 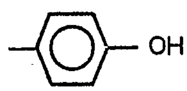 | 249 °C (EtoH / DMF) |
| 45 | 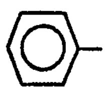 | 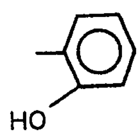 | 112 °C (EtoH) |
| 46 | 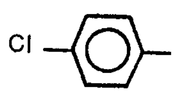 | 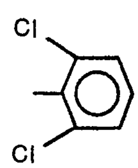 | 156 °C (EtoH / DMF) |
| 47 | 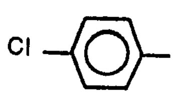 | 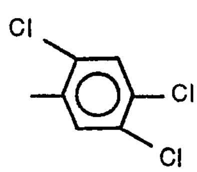 | 195 °C (DMF) |

| Beispiel Nr. | $R_1$ | $R_2$ | Fp. |
|---|---|---|---|
| 48 | 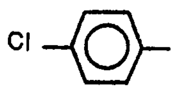 | 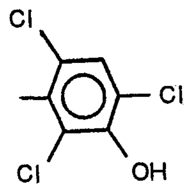 | 167 °C (EtoH) |
| 49 | 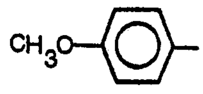 | 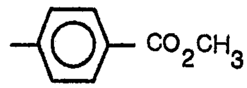 | 175 °C (DMF) |
| 50 | 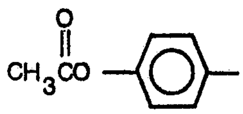 | 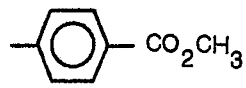 | 190 °C (DMF) |
| 51 | 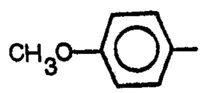 | $-CH(CH_3)_2$ | 1) |

1) Kp (0,27 mbar) = 136–38 °C.

Beispiel 52

18.42 g (0.1 Mol) Terephthaloylcyanid und 35.45 g (0.2 Mol) 4-Diäthylaminobenzaldehyd in 200 ml THF werden, wie unter Beispiel 1 beschrieben, umgesetzt.

Ausbeute: 21,82 g (37% d.Th.)
Bis-1,4-[4-chlor-2(4-diäthylaminophenyl)-5-oxazolyl]-benzol
gelbe Kristalle
Fp.: 272°C (DMF)

**Patentansprüche für die Vertragsstaaten: BE CH DE FR IT NL SE**

1. 4-Chloroxazole der allgemeinen Formel I bzw. II

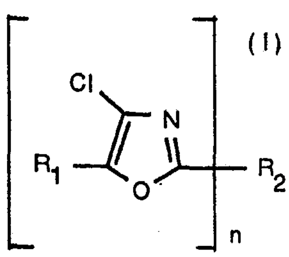 (I) 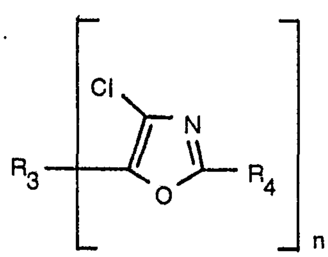 (II)

wobei n gleich 1 oder 2 ist, im Fall n gleich 1 $R_1$, $R_2$, $R_3$ und $R_4$ gleich oder verschieden sind und Phenyl oder Naphthyl, die durch eine bis drei Gruppen aus der Reihe Halogen, Cyano, Nitro, Trifluoromethyl, Amino, mono- oder di-1—2 C-Alkylamino, 1—4 C-Alkyl, 1—2 C-Alkoxy, Hydroxy, Carbo-1—3 C-alkoxy, Phenoxy, Styryl oder Chlorstyryl substituiert sein können, oder Pyrenyl oder einen N, O oder S enthaltenden 5 bis 6-gliedrigen Heterocyclus, der durch Chlor oder Methoxy-Gruppen substituiert sein oder einen ankondensierten Benzolring tragen kann, bedeuten, $R_2$ und $R_4$ zusätzlich $C_1$—$C_{12}$-Alkyl bedeuten, und im Fall n gleich 2 $R_1$ und $R_4$ die gleiche Bedeutung haben wie oben für den Fall n gleich 1

und $R_2$ und $R_3$ Phenylen oder Naphthylen bedeuten und wobei solche Verbindungen der Formel I ausgenommen sind, bei denen n gleich 1 ist, $R_1$ Phenyl und $R_2$ Phenyl, p-Methoxyphenyl, p-Methylphenyl, p-Nitrophenyl, p-Hydroxyphenyl oder p-Ethylphenyl oder $R_1$ p-Nitrophenyl und $R_2$ Phenyl darstellt.

2. Verbindungen nach Anspruch 1, wobei n gleich 1 oder 2 ist, im Fall n gleich 1, $R_1$, $R_2$, $R_3$ und $R_4$ Phenyl oder Naphthyl, die durch eine bis drei Gruppen aus der Reihe Fluor, Chlor oder Brom, Cyano, Nitro, Trifluoromethyl, Amino, mono- oder di-1—2 C-Alkylamino, 1—4 C-Alkyl, 1—2 C-Alkoxy, Hydroxy, Carbo-1—3 C-alkoxy, Phenoxy, Styryl substituiert sein können oder Furanyl, Benzofuranyl, Thienyl, Pyrimidyl, Benzthienyl oder Benzoxazolyl, die durch Chlor oder Methoxy substituiert sein können, bedeuten, und im Fall n gleich 2 $R_1$ und $R_4$ die gleiche Bedeutung haben wie zuvor für n gleich 1 angegeben, und $R_2$ und $R_3$ Phenylen bedeuten.

3. Verbindungen der allgemeinen Formel I bzw. II nach Anspruch 1, wobei n gleich 1 ist und $R_1$, $R_2$, $R_3$ und $R_4$ Di-1—2 C-alkylaminophenyl, Aminophenyl, Fluor-, Chlor- oder Bromphenyl, Trifluormethylphenyl oder 1—2 C-Alkoxyphenyl bedeuten.

4. Verfahren zur Herstellung der Verbindungen gemäß Anspruch 1, dadurch gekennzeichnet, daß man ein Benzoylcyanid der allgemeinen Formel III

$$R_1 - COCN \quad \text{bzw.} \quad R_3 - (COCN)_n \qquad \text{(III)}$$

mit einem Aldehyd der allgemeinen Formel IV

$$R_2 - (CHO)_n \quad \text{bzw.} \quad R_4 - CHO \qquad \text{(IV)}$$

umsetzt und aus dem dabei entstandenen Oxazol-Hydrochlorid durch Behandeln mit Wasser die 4-Chloroxazole der Formel I bzw. II freisetzt.

5. Verwendung der 4-Chloroxazole gemäß Anspruch 1 als optische Aufheller.

6. Verwendung der 4-Chloroxazole gemäß Anspruch 1 zur Herstellung photoleitender Schichten in der Elektrophotographie.

**Patentansprüche für den Vertragsstaat: AT**

1. Verfahren zur Herstellung von 4-Chloroxazolen der allgemeinen Formel I bzw. II

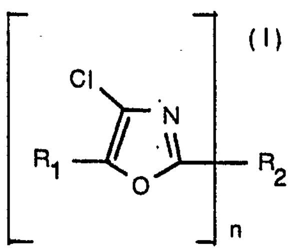
(I)

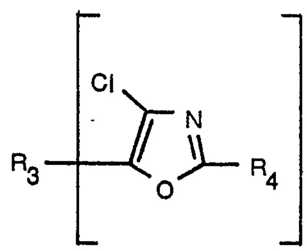
(II)

wobei n gleich 1 oder 2 ist, im Fall n gleich 1 $R_1$, $R_2$, $R_3$ und $R_4$ gleich oder verschieden sind und Phenyl oder Naphthyl, die durch eine bis drei Gruppen aus der Reihe Halogen, Cyano, Nitro, Trifluoromethyl, Amino, mono- oder di-1—2 C-Alkylamino, 1—4 C-Alkyl, 1—2 C-Alkoxy, Hydroxy, Carbo-1—3 C-alkoxy, Phenoxy, Styryl oder Chlorstyryl substituiert sein können, oder Pyrenyl oder einen N, O oder S enthaltenden 5 bis 6-gliedrigen Heterocyclus, der durch Chlor oder Methoxy-Gruppen substituiert sein oder einen ankondensierten Benzolring tragen kann, bedeuten, $R_2$ und $R_4$ zusätzlich $C_1$—$C_{12}$-Alkyl bedeuten, und im Fall n gleich 2 $R_1$ und $R_4$ die gleiche Bedeutung haben wie oben für den Fall n gleich 1 und $R_2$ und $R_3$ Phenylen oder Naphthylen bedeuten und wobei solche Verbindungen der Formel I ausgenommen sind, bei denen n gleich 1 ist, $R_1$ Phenyl und $R_2$ Phenyl, p-Methoxyphenyl, p-Methylphenyl, p-Nitrophenyl, p-Hydroxyphenyl oder p-Ethylphenyl oder $R_1$ p-Nitrophenyl und $R_2$ Phenyl darstellt, dadurch gekennzeichnet, daß man ein Benzoylcyanid der allgemeinen Formel III

$$R_1 - COCN \quad \text{bzw.} \quad R_3 - (COCN)_n \qquad \text{(III)}$$

mit einem Aldehyd der allgemeinen Formel IV

$$R_2 - (CHO)_n \quad \text{bzw.} \quad R_4 - CHO \qquad \text{(IV)}$$

umsetzt und aus dem dabei entstandenen Oxazol-Hydrochlorid durch Behandeln mit Wasser die 4-Chloroxazole der Formel I bzw. II freisetzt.

2. Verfahren nach Anspruch 1, dadurch gekennzeichnet, daß man eine Verbindung der Formel I bzw. II herstellt, wobei n gleich 1 oder 2 ist, im Fall n gleich 1 $R_1$, $R_2$, $R_3$ und $R_4$ Phenyl oder Naphthyl, die durch eine bis drei Gruppen aus der Reihe Fluor, Chlor oder Brom, Cyano, Nitro, Trifluormethyl, Amino, mono- oder di-1—2 C-Alkylamino, 1—4 C-Alkyl, 1—2 C-Alkoxy, Hydroxy, Carbo-1—3 C-alkoxy, Phenoxy, Styryl substituiert sein können, oder Furanyl, Benzofuranyl, Thienyl, Pyrimidyl, Benzthienyl oder Benzoxazolyl, die durch Chlor oder Methoxy substituiert sein können, bedeuten, und im Fall n gleich 2 $R_1$ und $R_4$ die gleiche Bedeutung haben, wie zuvor für n gleich 1 angegeben und $R_2$ und $R_3$ Phenylen bedeuten.

3. Verfahren nach Anspruch 1, dadurch gekennzeichnet, daß man eine Verbindung der Formel I bzw. II herstellt, wobei n gleich 1 ist und $R_1$, $R_2$, $R_3$ und $R_4$ Di-1—2 C-alkylaminophenyl, Aminophenyl, Fluor-, Chlor- oder Bromphenyl, Trifluormethylphenyl oder 1—2 C-Alkoxyphenyl bedeuten.

4. Verwendung der 4-Chloroxazole gemäß Anspruch 1 als optische Aufheller.

5. Verwendung der 4-Chloroxazole gemäß Anspruch 1 zur Herstellung photoleitender Schichten in der Elektrophotographie.

**Revendications pour les Etats contractants: BE CH DE FR IT NL SE**

1. 4-Chloroxazoles de formule générale I ou II,

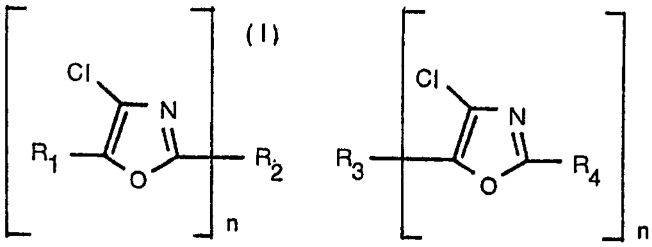

(I) (II)

dans lesquelles n est égal à 1 ou 2, dans le cas où n = 1, $R_1$, $R_2$, $R_3$ et $R_4$ sont identiques ou différents et représentent un groupe phényle ou naphtyle pouvant être substitués par un à trois groupes choisis parmi les suivants: halogène, cyano, nitro, trifluorométhyle, amino, mono-alkyl $C_{1-2}$-amino ou di-alkyl $C_{1-2}$-amino, alkyl $C_{1-4}$, alcoxy $C_{1-2}$, hydroxyle, carbo-alcoxy $C_{1-3}$, phénoxy, styryle ou chlorostyryle, ou un groupe pyrényle ou un hétérocycle à 5 ou 6 chaînons renfermant un atome de N, de O ou de S, pouvant être substitué par du chlore ou des groupes méthoxy ou bien porter un noyau benzénique condensé, $R_2$ et $R_4$ représentent en outre un groupe alkyle $C_{1-12}$ et dans le cas où n=2, $R_1$ et $R_4$ ont la même signification que dans le cas ci-dessus où n=1 et $R_2$ et $R_3$ sont le groupe phénylène ou naphtylène, les composés de formule I étant exceptés dans lesquels n=1, $R_1$ est phényle et $R_2$ phényle, p-méthoxyphényle, p-méthylphényle, p-nitrophényle, p-hydroxyphényle ou p-éthylphényle ou $R_1$ est p-nitrophényle et $R_2$ phényle.

2. Composés selon la revendication 1, dans lesquels n est égal à 1 ou 2, dans le cas où n=1, $R_1$, $R_2$, $R_3$ et $R_4$ représentent phényle ou naphtyle pouvant être substitué par un à trois groupes choisis parmi les suivants: le fluor, le chlore, ou le brome, le cyano, le nitro, le trifluorométhyle, l'amino, le mono-alkyl $C_{1-2}$-amino, le di-alkyl $C_{1-2}$-amino, l'alkyle $C_{1-4}$, l'alcoxy $C_{1-2}$, l'hydroxyle, le carbo-alcoxy $C_{1-3}$, le phénoxy, le styryle ou bien représentent un groupe furanyle, benzofuranyle, thiényle, pyrimidyle, benzothiényle ou benzoxazolyle éventuellement substitués par un groupe méthoxy ou un atome de chlore, et, dans le cas où n=2, $R_1$ et $R_4$ ont la signification indiquée ci-dessus pour n=1, et $R_2$ et $R_3$ représentent le phénylène.

3. Composés de formule générale I ou II selon la revendication 1, dans lesquels n=1 et $R_1$, $R_2$, $R_3$ et $R_4$ représentent un groupe di-alkyl $C_{1-2}$-aminophényle, aminophényle, fluorophényle, chlorophényle ou bromophényle, trifluorométhylephényle ou alcoxy $C_{1-2}$-phényle.

4. Procédé pour la préparation des composés selon la revendication 1, caractérisé en ce que l'on fait réagir un cyanure de benzoyle de formule générale III:

$$R_1—COCN \quad \text{ou} \quad R_3—(COCN)_n \qquad \text{(III)}$$

avec un aldéhyde de formule générale IV

$$R_2—(CHO)_n \quad \text{ou} \quad R_4—CHO \qquad \text{(IV)}$$

et on libère à partir du chlorhydrate d'oxazole ainsi formé les 4-chloroxazoles des formules I ou II par traitement par l'eau.

5. Utilisation des 4-chloroxazoles selon la revendication 1 comme azurants optiques.

6. Utilisation des 4-chloroxazoles selon la revendication 1 pour la fabrication de couches photoconductrices dans l'électrophotographie.

**Revendications pour l'Etat contractant: AT**

1. Procédé pour la préparation de 4-chloroxazoles de formule générale I ou II,

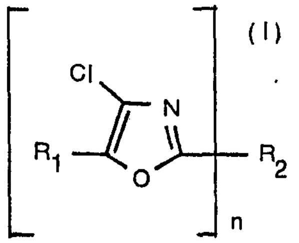

(I)

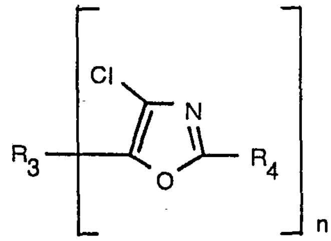

(II)

dans lesquelles n est égal à 1 ou 2, dans le cas où n = 1, $R_1$, $R_2$, $R_3$ et $R_4$ sont identiques ou différents et représentent un groupe phényle ou naphtyle pouvant être substitués par un à trois groupes choisis parmi les suivants: halogène, cyano, nitro, trifluorométhyle, amino, mono-alkyl $C_{1-2}$-amino ou di-alkyl $C_{1-2}$-amino, alkyl $C_{1-4}$, alcoxy $C_{1-2}$, hydroxyle, carbo-alcoxy $C_{1-3}$, phénoxy, styryle ou chlorostyryle, ou un groupe pyrényle ou un hétérocycle à 5 ou 6 chaînons renfermant un atome de N, de O ou de S, pouvant être substitué par du chlore ou des groupes méthoxy ou bien porter un noyau benzénique condensé, $R_2$ et $R_4$ représentent en outre un groupe alkyl $C_{1-12}$ et dans le cas où n=2, $R_1$ et $R_4$ ont la même signification que dans le cas ci-dessus où n=1 et $R_2$ et $R_3$ sont le groupe phénylène ou naphtylène, les composés de formule I étant exceptés dans lesquels n=1, $R_1$ est phényle et $R_2$ phényle, p-méthoxyphényle, p-méthylphényle, p-nitrophényle, p-hydroxyphényle ou p-éthylphényle ou $R_1$ est p-nitrophényle et $R_2$ phényle, caractérisé en ce que l'on fait réagir un cyanure de benzoyle de formule générale III:

$R_1$ — COCN ou (III)
$R_3$ — $(COCN)_n$

avec un aldéhyde de formule générale IV

$R_2$ — $(CHO)_n$ ou (IV)
$R_4$ — CHO

et on libère à partir du chlorhydrate d'oxazole ainsi formé les 4-chloroxazoles des formules I ou II par traitement par l'eau.

2. Procédé selon la revendication 1, caractérisé en ce que l'on prépare un composé de formule I ou II, dans lequel n est égal à 1 ou 2, dans le cas où n=1, $R_1$, $R_2$, $R_3$ et $R_4$ représentent phényle ou naphtyle pouvant être substitué par un à trois groupes choisis parmi les suivants: le fluor, le chlore ou le brome, le cyano, le nitro, le trifluorométhyle, l'amino, le mono-alkyl $C_{1-2}$-amino, le di-alkyl $C_{1-2}$amino, l'alkyl $C_{1-4}$, l'alcoxy $C_{1-2}$, l'hydroxyle, le carbo-alcoxy $C_{1-3}$, le phénoxy, le styryle ou bien représentent un groupe furanyle, benzofuranyle, thiényle, pyrimidyle, benzothiényle ou benzoxazolyle éventuellement substitués par un groupe méthoxy ou un atome de chlore, et, dans le cas où n=2, $R_1$ et $R_4$ on la signification indiquée ci-dessus pour n=1, et $R_2$ et $R_3$ représentent le phénylène.

3. Procédé selon la revendication 1, caractérisé en ce que l'on prépare un composé de formule I ou II, dans lequel n=1 et $R_1$, $R_2$, $R_3$ et $R_4$ représentent un groupe di-alkyl $C_{1-2}$-aminophényle, aminophényle, fluorophényle, chlorophényle ou bromophényle, trifluorométhylphényle ou alcoxy $C_{1-2}$-phényle.

4. Utilisation des 4-chloroxazoles préparés selon la revendication 1 comme azurants optiques.

5. Utilisation des 4-chloroxazoles préparés selon la revendication 1 pour la fabrication de couches photoconductrices dans l'electrophotographie.

**Claims for the Contracting States: BE CH DE FR IT NL SE**

1. 4-Chlorooxazoles of the general formula I or II

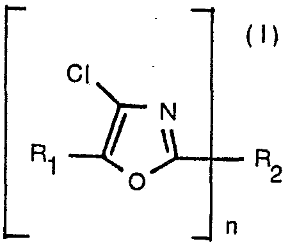 (I)

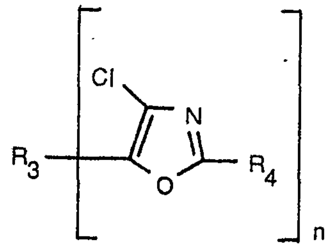 (II)

in which n denotes 1 or 2 and, in the case of n being 1, $R_1$, $R_2$, $R_3$ and $R_4$, which are identical or different, denote phenyl or naphthyl optionally substituted by 1 to 3 radicals selected from the group consisting of halogen, cyano, nitro, trifluoromethyl, amino, mono- or di-$C_1$—$C_2$ alkylamino, $C_1$—$C_4$ alkyl, $C_1$—$C_2$ alkoxy, hydroxy, $C_1$—$C_3$ alkoxycarbonyl, phenoxy, styryl, or chlorostyryl, or denote pyrenyl or a 5- or 6-membered heterocycle containing N, O or S which may be substituted by chlorine or by methoxy groups or may carry a fused benzene nucleus; $R_2$ and $R_4$ additionally denote $C_1$—$C_{12}$alkyl and in the case of n being 2, $R_1$ and $R_4$ have the same meaning as defined above for n being 1 and $R_2$ and $R_3$ denote phenylene or naphthylene, with the exception of those compounds of the formula I in which n is 1, $R_1$ denotes phenyl and $R_2$ denotes phenyl, p-methoxyphenyl, p-methylphenyl, p-nitrophenyl, p-hydroxyphenyl or p-ethylphenyl or $R_1$ denotes p-nitrophenyl and $R_2$ denotes phenyl.

2. Compounds as claimed in claim 1, wherein n denotes 1 or 2 and, in the case of n being 1, $R_1$, $R_2$, $R_3$ and $R_4$ denote phenyl or naphthyl optionally substituted by 1 to 3 radicals selected from the group consisting of fluorine, chlorine or bromine, cyano, nitro, trifluoromethyl, amino, mono- or di-$C_1$—$C_2$alkylamino, $C_1$—$C_4$alkyl, $C_1$—$C_2$alkoxy, hydroxy, $C_1$—$C_3$alkoxycarbonyl, phenoxy or styryl or denote furanyl, benzofuranyl, thienyl, pyrimidyl, benzothienyl or benzooxazolyl optionally substituted by chlorine or by methoxy groups and, in the case of n being 2, $R_1$ and $R_4$ have the same meaning as indicated for n being 1 and $R_2$ and $R_3$ denote phenylene.

3. Compounds of the general formula I or II as claimed in claim 1, wherein n denotes 1 and $R_1$, $R_2$, $R_3$ and $R_4$ denote di-$C_1$—$C_2$alkylaminophenyl, aminophenyl, fluoro, chloro or bromophenyl, trifluoromethylphenyl or $C_1$—$C_2$alkoxyphenyl.

4. Process for the preparation of compounds as claimed in claim 1, which comprises reacting a benzoyl cyanide of the general formula III

$$R_1 — COCN \quad \text{or} \qquad (III)$$
$$R_3 — (COCN)_n$$

with an aldehyde of the general formula IV

$$R_2 — (CHO)_n \quad \text{or} \qquad (IV)$$
$$R_4 — CHO$$

and liberating from the oxazole hydrochloride formed, the 4-chlorooxazoles of the formula I or II by a treatment with water.

5. Use of the 4-chlorooxazoles as claimed in claim 1 as optical brighteners.

6. Use of the 4-chlorooxazoles as claimed in claim 1 for the preparation of photoconductive layers in electrophotography.

**Claims for the Contracting State: AT**

1. Process for the preparation of 4-chlorooxazoles of the general formula I or II

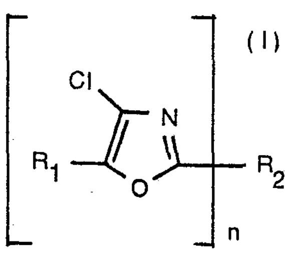 (I)

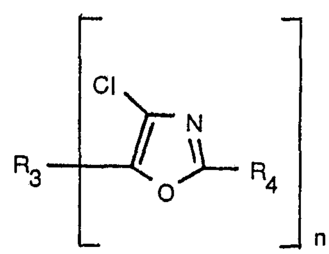 (II)

in which n denotes 1 or 2 and, in the case of n being 1, $R_1$, $R_2$, $R_3$ and $R_4$, which are identical or different, denote phenyl or naphthyl optionally substituted by 1 to 3 radicals selected from the group consisting of halogen, cyano, nitro, trifluoromethyl, amino, mono- or di-$C_1$—$C_2$ alkylamino, $C_1$—$C_4$ alkyl, $C_1$—$C_2$ alkoxy, hydroxy, $C_1$—$C_3$ alkoxycarbonyl, phenoxy, styryl, or chlorostyryl, or denote pyrenyl or a 5- or 6-membered heterocycle containing N, O or S which may be substituted by chlorine or by methoxy groups or may carry a fused benzene nucleus; $R_2$ and $R_4$ additionally denote $C_1$—$C_{12}$alkyl and, in the case of n being 2, $R_1$ and $R_4$ have the same meaning as defined above for n being 1 and $R_2$ and $R_3$ denote phenylene or naphthylene, with the exception of those compounds of the formula I in which n is 1, $R_1$ denotes phenyl and $R_2$ denotes phenyl, p-methoxyphenyl, p-methylphenyl, p-nitrophenyl, p-hydroxyphenyl or p-ethylphenyl or $R_1$ denotes p-nitrophenyl and $R_2$ denotes phenyl, which comprises reacting a benzoyl cyanide of the general formula III

$$R_1 — COCN \quad \text{or} \qquad (III)$$
$$R_3 — (COCN)_n$$

with an aldehyde of the general formula IV

$$R_2 — (CHO)_n \quad \text{or} \qquad (IV)$$
$$R_4 — CHO$$

and liberating from the oxazole hydrochloride formed, the 4-chlorooxazoles of the formula I or II by a treatment with water.

2. A process as claimed in claim 1, wherein a compound according to formula I or II is prepared, in which n denotes 1 or 2 and, in the case of n being 1, $R_1$, $R_2$, $R_3$ and $R_4$ denote phenyl or naphthyl optionally substituted by 1 to 3 radicals selected from the group consisting of fluorine, chlorine or bromine, cyano, nitro, trifluoromethyl, amino, mono- or di-$C_1$—$C_2$alkylamino, $C_1$—$C_4$alkyl, $C_1$—$C_2$alkoxy, hydroxy, $C_1$—$C_3$alkoxycarbonyl, phenoxy or styryl or denote furanyl, benzofuranyl, thienyl, pyrimidyl, benzothienyl or benzooxazolyl optionally substituted by chlorine or by methoxy groups and, in the case of n being 2, $R_1$ and $R_4$ have the same meaning as indicated for n being 1 and $R_2$ and $R_3$ denote phenylene.

3. A process as claimed in claim 1, wherein a compound according to formula I or II is prepared in which n denotes 1 and $R_1$, $R_2$, $R_3$ and $R_4$ denote di-$C_1$—$C_2$alkylaminophenyl, aminophenyl, fluoro, chloro or bromophenyl, trifluoromethylphenyl or $C_1$—$C_2$alkoxyphenyl.

4. Use of the 4-chlorooxazoles prepared as claimed in claim 1 as optical brighteners.

5. Use of the 4-chlorooxazoles prepared as claimed in claim 1 for the manufacture of photoconductive layers in electrophotography.